(19) Europäisches Patentamt / European Patent Office / Office européen des brevets

(11) **EP 3 367 963 B1**

(12) **EUROPEAN PATENT SPECIFICATION**

(45) Date of publication and mention of the grant of the patent:
**23.08.2023 Bulletin 2023/34**

(21) Application number: **16794834.8**

(22) Date of filing: **27.10.2016**

(51) International Patent Classification (IPC):
***A61C 19/06*** (2006.01)

(52) Cooperative Patent Classification (CPC):
**A61C 19/066**

(86) International application number:
**PCT/US2016/059004**

(87) International publication number:
**WO 2017/075142 (04.05.2017 Gazette 2017/18)**

(54) **TOOTH WHITENING AGENT**

ZAHNBLEICHMITTEL

AGENT DE BLANCHIMENT DES DENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2015 US 201514925457**

(43) Date of publication of application:
**05.09.2018 Bulletin 2018/36**

(73) Proprietor: **Colgate-Palmolive Company New York, NY 10022 (US)**

(72) Inventors:
• **JOHANSSON, Patrik**
  **Hoboken, New Jersey 07030 (US)**
• **LAVENDER, Stacey**
  **Chesterfield, New Jersey 08515 (US)**
• **DEMAREST, Scott**
  **Piscataway, NJ 08854 (US)**
• **ADAMS, Richard**
  **South Orange, New Jersey 07079 (US)**
• **BOYD, Thomas**
  **Metuchen, New Jersey 08840 (US)**
• **PATEL, Madhusudan**
  **Piscataway, NJ 08854 (US)**

(74) Representative: **Wibbelmann, Jobst**
**Wuesthoff & Wuesthoff**
**Patentanwälte PartG mbB**
**Schweigerstrasse 2**
**81541 München (DE)**

(56) References cited:
WO-A1-2008/112657   WO-A1-2013/155632
WO-A1-2015/034911   US-A1- 2015 064 645

**Description**

**BACKGROUND**

There are many approaches to whitening teeth, ranging from using high abrasive toothpaste to in-office dentist visits for power bleaching. Typically, removing both extrinsic and intrinsic stains require chemical bleaching.

Chemical bleaching includes a whitening agent that typically comprises an oxidizer. Oxidizers are applied to teeth for an amount of time before a user's teeth display the desired amount of whitening. Various parameters related to oxidizer agent, such as contact time and number of applications, the affect the amount of whitening.

Previous attempts to improve tooth whitening using a whitening agent have included exposing teeth to light power. However, these attempts require high irradiance densities (at least 200 mW/cm$^2$) and whitening agents that have high concentrations of oxidizer (at least 35 wt. % based on the total weight of the whitening agent). Although the previous attempts may have provided whiter teeth, using such high irradiance density and high concentrations of oxidizer results in increased tooth sensitivity and oral discomfort.

There would be an advantage to increasing the efficiency of the whitening process without needing to increase the irradiance density or the concentration of oxidizer. Increasing the efficiency of the whitening process would increase the overall whitening effect of a single treatment without having a corresponding increase in tooth sensitivity or soft tissue irritation. US 2015/064645 A1 discloses a tooth whitening agent comprising hydrogen peroxide in a concentration ranging from 0.1 to 38 percent by weight, used with a blue light at wavelength in the 400-500 nm range and at an irradiance of between 3.5 to about 20 mW/cm$^2$.

**BRIEF SUMMARY**

The invention pertains to a tooth whitening agent comprising hydrogen peroxide in a concentration ranging from 2 wt. % to 10 wt. % based on the total weight of the tooth whitening agent for use in a method of whitening teeth comprising: a) applying a tooth whitening agent to the tooth, for a pretreatment prior to b) continuously irradiating a tooth with light generated by a light source for an irradiation time period, which ranges from 60 seconds to 1800 seconds, the light having a wavelength that ranges from 400 nm to 410 nm and the light source emitting the light at an irradiance density ranging from 3 mW/cm2 to 10.0 mW/cm2 , as defined in claim 1.

In a preferred embodiment, the wavelength is 405 nm.

In a preferred embodiment, step b) is performed while the tooth whitening agent is on the tooth.

In a preferred embodiment, the whitening agent is substantially free of zinc oxide.

In a preferred embodiment, the hydrogen peroxide is present in a concentration ranging from 3 wt. % to 6 wt. % based on the total weight of the whitening agent.

Further embodiments are defined by the appended dependent claims 6-10.

**BRIEF DESCRIPTION OF THE DRAWINGS**

The present invention will become more fully understood from the detailed description and the accompanying drawings, wherein:

Figure 1 is a graphical representation of coffee stain bleaching data generated according to one embodiment of the present invention;

Figure 2 is a graphical representation of tea stain bleaching data generated according to one embodiment of the present invention;

Figure 3 is a graphical representation of wine bleaching data generated according to one embodiment of the present invention;

Figure 4 is a graphical representation of stain bleaching data for a treatment period of 5 minutes according to one embodiment of the present invention;

Figure 5 is a graphical representation of stain bleaching data for a treatment period of 10 minutes according to one embodiment of the present invention; and

Figure 6 is a graphical representation of stain bleaching data superimposed with bovine teeth whitening data according to one embodiment of the present invention.

Figure 7 is a graphical representation of whitening data generated according to one embodiment of the present invention;

## DETAILED DESCRIPTION

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range.

Teeth stains may arise from discoloration in the enamel component and/or dentin component of a tooth. The dentin component is a calcified tissue that is typically covered by the enamel component of a crown. Through the translucency of the enamel, changes in color of the dentin component can be seen from outside of the tooth. Therefore, to effectively whiten and remove stains from a tooth, the whitening method of the present invention may include changing the color of not only the enamel component of a tooth, but also the underlying dentin component of a tooth. However, when using a light to whiten the enamel component and the dentin component of a tooth, the light may scattering as it passes through a tooth. With increased light scattering there may be a corresponding decrease in effectiveness in whitening. Furthermore, the present invention includes a method for whitening teeth that not only result in superior whitening but also reduced risk of increased mouth sensitivity and damage to the user's teeth and gums. According to some embodiments, the method of the present invention may include a whitening treatment that includes light emitted from a light source used in combination with a whitening agent.

The whitening treatment of the present invention may include a number parameters including, but not limited to: the type of light source; the wavelength of the light that is emitted from the light source; irradiance density ($mW/cm^2$) of the light; the distance from the exterior surface of the light source and the tooth surface; and the amount of time the tooth is irradiated by the light from the light source ("irradiation time period"). Additionally, the parameters of the present invention may include: whitening agent composition (including the type of active agent in the whitening agent); the concentration of active ingredient in the whitening agent; and an amount of time a tooth may be pretreated with the whitening agent prior to being exposed to irradiating from the light source ("pretreatment time period").

The light source of the present invention may include at least one LED, Xenon lamp, electroluminescence, or a combination thereof. The light source may comprise a plurality of LEDs. In some non-limiting embodiments, the LED may be a printed inorganic LED, a micro conventional LED, organic LED (OLED), or a combination thereof. The light source may include a single LED or a plurality of individual LEDs.

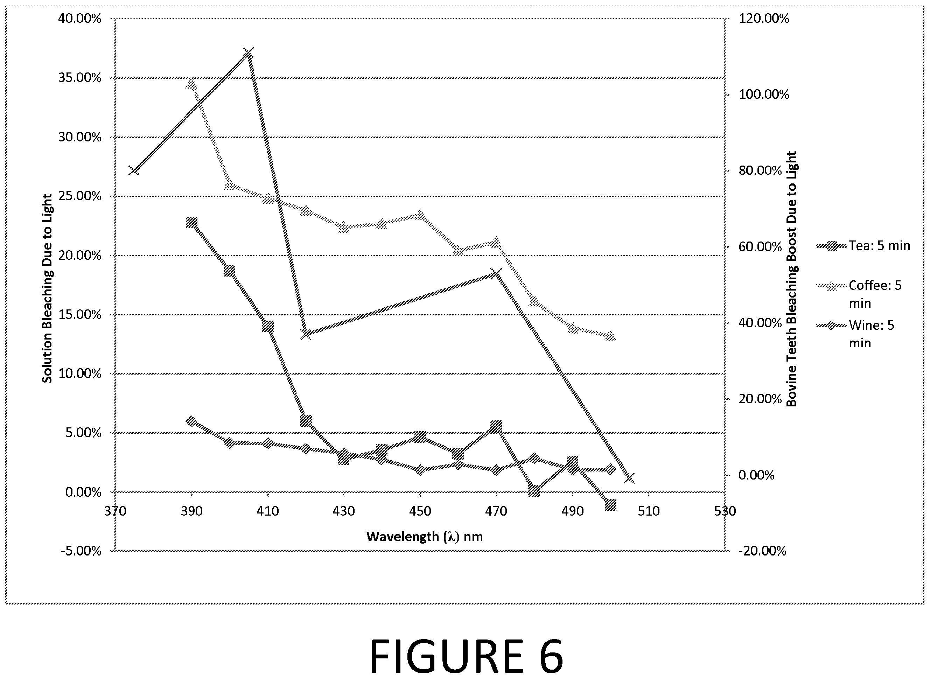

The wavelength of the light emitted by the light source may range from 375 nm to 505 nm - including all sub-ranges and integers there-between. In theory, certain stains can be effectively bleached using light within the ultra violet (UV) spectrum (i.e. less than about 400 nm). However, as wavelength of the light becomes shorter within the UV spectrum, there is a greater risk of damaging soft tissue within the user's mouth. Additionally, using wavelengths that are less than about 400 nm may create difficulties in producing effective tooth whitening due to the scattering of incident light on the tooth as the light penetrates into the tooth - as demonstrated by Figure 6, discussed herein. For this reason, the wavelength of the light that is emitted by the light source is preferably at least 390 nm.

The light source of the present invention may emit light having a wavelength that ranges from 390 to 500 nm - including all sub-ranges and integers there-between. In some embodiments, the light source may emit light having a wavelength that ranges from 390 to 420 nm - preferably from 400 nm to 410 nm - including all sub-ranges and integers there-between.

The light source of the present invention may emit light having a wavelength of 400 nm, 405 nm, or 410 nm. In an alternative embodiment, the wavelength of light emitted by the light source may range from 390 nm to 450 nm - including all sub-ranges and integers there-between.

The present disclosure provides an example of a method of whitening teeth and bleaching stains at a low irradiance density - i.e. less than about 50 $mW/cm^2$. The term "about" means +/- 5%. According to some embodiments of the present invention, the irradiation density may include an emission irradiance density and an incident irradiance density. The emission irradiance density is the irradiance density of the light when it is emitted from the LED light source. The emission irradiance density may be measured at the exterior surface of the LED light source. The incident irradiance density is the irradiance density of the light when it reaches the tooth. The incident irradiance density may be measured at the tooth surface.

Irradiance density is a measure of light intensity. Light intensity has an inverse squared relationship to distance ($d^{-2}$) ("irradiance distance" - i.e. the distance between the light source and the tooth). Thus, as the irradiance distance increases, the incident irradiance density becomes smaller relative to the emitted irradiance density. According to some embodiments of the present invention, the irradiance distance may range from about 0 mm to about 12 mm - alternatively about 2 mm to 10 mm - including all sub-ranges and integers there-between. The incident irradiance density of the light may be 1% to 99% - including all sub-ranges there-between - of the emission irradiance density depending on the irradiance distance.

The decrease in incident irradiance density from emission irradiance density that occurs as light travels the irradiance distance may be mitigated by changing the medium in which the light travels across the irradiance distance.

The medium may be air, saliva, a whitening composition, or a combination thereof. In a preferred embodiment, the light transmittance through the whitening agent according to the present invention ranges from about 7% to about 9% at a irradiance distance of about 2 mm.

The emission irradiance density may range from about 3 mW/cm$^2$ to about 10 mW/cm$^2$ - preferably from about 4 W/cm$^2$ to about 8.1 mW/cm$^2$. Surprisingly, a plateau in whitening has been discovered for light having an emission irradiation density greater of about 8 mW/cm$^2$ or greater at wavelengths ranging from 390 nm to 420 nm, as discussed herein. Thus, according to the present invention, it has been discovered that superior tooth whitening can be achieved at a wavelength ranging from 400 nm to 410 nm (including all sub-ranges and integers there-between) - without necessitating high irradiance density (i.e. greater than 20 mW/cm$^2$) to achieve the desired tooth whitening effect. The result is a tooth whitening method that reduces damage and sensitivity to the user's teeth and gums.

The light source of the present invention is continuous. When continuously irradiating a tooth, the emission irradiance density remains substantially constant. According to the present invention, the phrase "substantially constant" means fluctuations less than 2%.

Pulsating light may cycle between a maximum and a minimum pulsating emission irradiance density. The cycling may occur at a frequency ranging from 500 Hz to 2,000 Hz - including all sub-ranges and integers there-between. In an example which is not part of the present invention, the pulsating light source cycles at a frequency of about 1,000 Hz. The pulsating light may cycle ON and OFF - i.e., the maximum pulsating irradiance density may be 100% of the emission irradiance density, and the minimum pulsating irradiance density may be 0% of the emission irradiance density. Oscillating between ON and OFF results in a power load of about 50% as the 100% and 0% are averaged together.

According to other examples which are not part of the present invention, the maximum pulsating irradiance density that is 100% of the emission irradiance density and the minimum pulsating irradiance density is less than the maximum pulsating irradiance density but also a non-zero percentage of the maximum pulsating density. Suitable non-limiting examples of minimum pulsating irradiance density is about 1% to about 75% of the emission irradiance density, alternatively from about 1% to about 50%; alternatively from about 1% to about 25%; alternatively from about 1% to about 10% - including all sub-ranges and integers there-between. According to these examples, the power load of the pulsating light may range from greater than 50% to less than 100% - including all sub-ranges there-between.

The whitening method further includes an irradiation time period that is an amount of time that the tooth is irradiated by the light at the desired irradiation distance. The irradiation time period may range from about 60 seconds to about 1800 seconds - including all sub-ranges and individual time periods there between. According to some embodiments, the irradiation time period may range from about 60 seconds to about 900 seconds - including all sub-ranges and integers there-between. In a preferred embodiment, the irradiation time period may range from about 300 seconds minutes to about 600 seconds - including all sub-ranges there between. The irradiation time period may depend on a number of other whitening parameters - including irradiance density and oxidizing parameters, as discussed herein.

According to the present invention, once the irradiation time period expires, the tooth being treated may cease to be irradiated by the light emitted from the light source, thereby completing a single whitening treatment. The irradiation time period may be measured by an automatic timer that automatically turns OFF the light source at the expiration of the irradiation time period. In other embodiments, at the expiration of the irradiation time period, an indicator provided by a device comprising the light source may notify the user (by sound, vibration, etc.) to remove the light source from oral cavity, wherein the user may manually turn OFF the light source.

After the irradiation time period expires, the whitening method may further include a recovery time period where the tooth is not irradiated by the light from the light source. The recovery time period may range from about 4 hours to about 96 hours - including all sub-ranges and integers there-between. The single treatment may be repeated a plurality of times ranging from 2 to 10 single treatments. In some methods, the whitening method includes 8 or fewer single treatments. In other embodiments, the present invention provides for an intermediate tooth whitening treatment wherein a tooth is whitened by only a single treatment within a time period of at minimum 7 days. The intermediate tooth whitening may be useful as preparation for social gatherings or professional events.

According to some methods, each treatment may further comprise a whitening agent that is used in combination with the light source during the whitening process. Non-limiting examples of whitening agent may include an oxidizer, such as carbamide peroxide, calcium peroxide, zinc peroxide, hydrogen peroxide, and mixtures thereof. In a preferred embodiment, the whitening agent may comprise hydrogen peroxide ($H_2O_2$).

According to the present invention, the whitening agent comprises $H_2O_2$ in a concentration ranging from about 3 wt. % to about 10 wt. % based on the total weight of the whitening composition. In a preferred embodiment, the whitening agent comprises $H_2O_2$ in a concentration ranging of about 9 wt. % based on the total weight of the whitening composition. In a preferred embodiment, the whitening agent comprises $H_2O_2$ in a concentration ranging of about 6 wt. % based on the total weight of the whitening composition. In some embodiments, the whitening agent may comprise about 4.5 wt. % of $H_2O_2$ based on the total weight of the whitening composition. In some embodiments, the whitening agent may comprise about 3 wt. % of $H_2O_2$ based on the total weight of the whitening composition.

The whitening composition may include additional components, such as a carrier and a therapeutic agent. Non-limiting examples of carrier include water, alcohol, gelling-polymer, or the like. Non-limiting examples of therapeutic agent may include a hard tissue anesthetic, a soft tissue anesthetic, a fluoride, an antiviral medicament, anti-tartar agent, a halitosis agent, a salivary flow agent, an antibiotic, or an antimicrobial agent.

Non-limiting examples of therapeutic agent comprise zinc particles - e.g., zinc oxide. The particle size of the therapeutic agent is selected such that the therapeutic agent provides therapeutic effects to the oral cavity without acting as photocatalyst for the whitening agent. In a preferred embodiment, the therapeutic agent comprises zinc oxide particles having a particle size that imparts no photocatalytic activity on hydrogen peroxide gel present in the whitening composition. In a preferred embodiment, the whitening composition is substantially free of particles that impart photocatalytic activity to the whitening agent. According to the present invention, the term "substantially free" constitutes less than 0.005 wt. % based on the total weight of the referenced composition. The whitening agent may be a low viscosity liquid or a high viscosity paste.

Each single method may include (1) applying the whitening agent to the oral cavity - i.e. onto one or more teeth; and (2) irradiating the one or more teeth with the light from the light source for the irradiation time period; and (3) ceasing to irradiate the one or more teeth with the light from the light source.

The application of the whitening agent to a user's tooth may be aided by a dispenser. In some non-limiting embodiments, the dispenser may be a brush, syringe, or mouth insert (e.g. a filled bladder). In some embodiments, steps (1) and (2) may occur simultaneously. In other embodiments, step (2) occurs subsequent to step (1) after a pretreatment time period.

After the whitening agent is applied to one or more teeth, there may be a pretreatment time period that spans from the application of the whitening agent to when the one or more teeth are irradiated by the light from the light source. The pretreatment time period may range from about 60 seconds to about 15 minutes - including all sub-ranges there between. Upon expiration of the pretreatment time period, the light source is activated and the one or more teeth are irradiated with light from the light source for the irradiation time period.

The pretreatment time period may ensure that the whitening agent is sufficiently disbursed throughout the oral cavity, thereby ensuring each whitening treatment reaches the maximum amount of potential whitening. After completing step (2), which includes irradiating the tooth surface with the light from the light source for the irradiation time period, the light source may be turned off and removed from the oral cavity - thereby completing a single treatment.

According to one embodiment of the present invention, teeth in an oral cavity can be whitened by irradiating the teeth with a continuous light emitted from an LED light source, the light source emitting the light at an emission irradiance density level that is less than or equal to 10 mW/cm$^2$, and the light having a wavelength of about 400 nm to 410 nm. The light source may be operated in combination with a whitening agent that comprises hydrogen peroxide in a concentration that ranges from about 3 wt. % to about 9 wt. % for an irradiation time period ranging from about 5 minutes to about 20 minutes. Under these conditions, the irradiance density levels of the current invention help achieve tooth whitening at lower power levels.

According to other embodiments, the present invention is directed to a whitening agent for use in a method of bleaching a stain created by a stain composition. Non-limiting examples of stain compositions include coffee, tea, wine, or tobacco. Certain stain composition may contain polyphenols that give rise to darkness that may be lightened during bleaching.

Molecules that are capable of producing stains include polyphenols. Some non-limiting examples of polyphenols include thearubigin, theaflavin (TF), anthocyanins, flavanols, flavonols, and hydroxycinnamic acids. Thearubigins and theaflavins can be found in dark teas and anthocyanins, flavanols, flavonols, and hydroxycinnamic acids may be found in red wines.

Anthocyanidins may include compounds such as aurantindin, cyanidin, delphinidin, europinidin, pelargonidin, malvidin, peonidin, petunidin, and rosinidin. Flavanols include compounds that are derivatives of flavans and include compounds such as catechin, epicatechin gallatae, epigallocatechin, epigallocatechin gallate, and proanthocyanidins.

Flavonols may include compounds such as 3-Hydroxyflavone (3-hydroxy-2-pheylchormen-4-one), azaleatin (2-(3,4-dihydroxyphenyl)-3,7-dihydroxy-5-methoxychromen-4-one), fisetin (3,3',4',7-tetrahydroxy-2-phenylchromen-4-one), galangin (3,5,7-trihydroxy-2-phenylchromen-4-one), gossypetin (2-(3,4-dihydroxyphenyl)-3,5,7,8-tetrahydroxychromen-4-one), kaempferide (3,5,7-trihydroxy-2-(4-methoxyphenyl)chromen-4-one), kaempferol (3,4',5,7-tetrahydroxy-2-phenylchromen-4-one), isorhamnetin (3,5,7-trihydroxy-2-(4-hydroxy-3-methoxyphenyl)chromen-4-one), morin (2-(2,4-dihydroxyphenyl)-3,5,7-trihydroxychromen-4-one), myricetin (3,3',4',5',5,7-hexahydroxy-2-phenylchromen-4-one), matsudaidain (2-(3,4-dimethoxyphenyl)-3-hydroxy-5,6,7,8-tetramethoxychromen-4-one), pachypodol (5-hydroxy-2-(4-hydroxy-3-methoxyphenyl)-3,7-dimethoxychromen-4-one), quercetin (3,3',4',5,7-pentahydroxy-2-phenylchromen-4-one), rhamnazin (3,5-dihydroxy-2-(4-hydroxy-3-methoxyphenyl)-7-methoxychromen-4-one), rhamnetin (2-(3,4-dihydroxyphenyl)-3,5-dihydroxy-7-methoxychromen-4-one), and combinations thereof.

Hydoxycynnamic acid include compounds such as α-cyano-4-hydroxycinnamic acid, caffeic acid, cichoric acid, cinnamic acid, chlorogenic acid, diferulic acids, coumaric acid, coumarin, ferulic acid (3-methoxy-4-hydroxycinnamic acid), sinapinic acid (3,5-dimethoxy-4-hydroxycinnamic acid or sinapic acid), and combinations thereof.

According to some embodiments, the present invention includes a whitening agent for use in a method for bleaching a coffee stain. The method may include one or more treatments of irradiating a coffee stain with light emitted from an LED light source, wherein the light has a wavelength that ranges from 400 nm to 410 nm and the light source emits the light at an irradiance density ranging from 3 mW/cm$^2$ to 10.0 mW/cm$^2$. In some embodiments, the treatment may include emitting light from the LED light source for an interval spanning up to 600 seconds at a wavelength of 400 nm to 410 nm. In some embodiments, the method of bleaching the coffee stain may include repeating the treatment 2 to 8 times - including all integers there between.

According to some embodiments, the present invention includes a whitening agent for use in a method for bleaching a tea stain. The method may include one or more treatments of irradiating a tea stain with light emitted from an LED light source, wherein the light has a wavelength that ranges from 400 nm to 410 nm and the light source emits the light at an irradiance density ranging from 3 mW/cm$^2$ to 10.0 mW/cm$^2$. In some embodiments, the tea stain may be irradiated with the light from the LED light source for a period up to 500 seconds and the wavelength of the light is 400 to 410 nm.

Some embodiments of the present invention may include a whitening agent for use in a method of bleaching a wine stain. The method may include irradiating a wine stain with a light emitted from an LED light source, wherein the light has a wavelength that ranges from 400 nm to 410 nm and the light source emits the light an irradiance density ranging from 3 W/cm$^2$ to 10.0 mW/cm$^2$.

Unless otherwise specified, all percentages and amounts expressed herein and elsewhere in the specification should be understood to refer to percentages by weight. The amounts given are based on the active weight of the material.

**EXAMPLES**

The examples of the present invention include color measurements before and after the whitening process of the present invention. Specifically, the L*a*b* color space of each tooth is measured prior to any treatments as well as after each treatment. The L*a*b* color space values are used to calculated the increase in whiteness ($\Delta$W) using the following calculation:

$$W^* = [(L^*-100)^2 + (a^*)^2 + (b^*)^2]^{1/2}$$

$$\Delta W = W^*\text{post-treatment} - W^*\text{baseline}$$

wherein the W*post-treatment is the measured W* value after whitening treatment and W*baseline is the W* value before treatment. Using the above referenced calculation, the more negative the $\Delta$W value is, the greater the whitening effect. All experiments were performed at room temperature (25 °C - 27 °C).

Example 1

Example 1 includes whitening bovine teeth according to the whitening method of the present invention. Specifically, the bovine teeth were whitened according to a plurality of whitening treatments - each treatment included a whitening agent comprising 4.5 wt. % of $H_2O_2$ and irradiating a tooth surface with light from an LED for a period of 15 minutes, wherein the light at the surface of the LED had a irradiance density of 4 mW/cm$^2$. The light included a plurality of wavelengths ($\lambda$).

**Table 1**

| Treatment # | No Light | ($\lambda$) 375 nm | ($\lambda$) 405 nm | ($\lambda$) 420 nm | ($\lambda$) 470 nm | ($\lambda$) 505 nm |
|---|---|---|---|---|---|---|
| 1 | -2.02 | -4.14 | -5.44 | -2.27 | -3.23 | -1.91 |
| 2 | -2.91 | -5.73 | -6.87 | -3.96 | -4.83 | -2.46 |
| 3 | -3.64 | -7.12 | -8.68 | -5.07 | -6.09 | -3.33 |
| 4 | -4.30 | -7.94 | -9.90 | -6.37 | -6.60 | -3.95 |
| 5 | -4.71 | -9.24 | -10.72 | -6.73 | -7.17 | -4.61 |
| 6 | -5.18 | -9.88 | -11.57 | -7.04 | -8.03 | -5.27 |
| 7 | -5.64 | -10.16 | -11.90 | -7.73 | -8.62 | -5.58 |

As demonstrated by Table 1, whitening treatment using a combination of $H_2O_2$ and light from the LED resulted in superior whitening of the bovine teeth as compared to whitening treatment with $H_2O_2$ without light. Furthermore, although lower wavelengths would be expected to result in superior whitening, light having a wavelength of 405 nm out-performed the whitening that used light having a wavelength of 375 nm. A partial reason for this may be due to the fact light having a wavelength in UV spectrum result in greater scattering as it enters the tooth, and, therefore, fails to perform as well as light in the visible spectrum. The whitening treatment performance of the various light sources used in combination with H2O2 compared to H2O2 used with no light can be summarized in Table 2

**Table 2**

|  | $(\lambda)$ 375 nm | $(\lambda)$ 405 nm | $(\lambda)$ 420 nm | $(\lambda)$ 470 nm | $(\lambda)$ 505 nm |
|---|---|---|---|---|---|
| % Change in $\Delta W$ | -80 | -111 | -37 | -53 | 0.9 |

Example 2

Example 2 demonstrates the difference in irradiance density for whitening treatment. Specifically, bovine teeth were whitened according to a plurality of whitening treatments - each treatment included a whitening agent comprising 4.5 wt. % of $H_2O_2$ and irradiating a tooth with light from an LED for a period of 15 minutes, wherein the light having a wavelength of 405 nm. The light included a plurality of irradiance densities ($mW/cm^2$).

**Table 3**

| Treatment # | No Light | 0.34 $mW/cm^2$ | 1.6 $mW/cm^2$ | 4.1 $mW/cm^2$ | 8.1 $mW/cm^2$ |
|---|---|---|---|---|---|
| 1 | -2.1 | -3.0 | -3.8 | -5.8 | -4.4 |
| 2 | -2.9 | -4.0 | -5.7 | -7.0 | -9.1 |
| 3 | -3.8 | -4.4 | -6.4 | -8.8 | -10.0 |
| 4 | -4.4 | -5.1 | -7.7 | -10.0 | -12.5 |
| 5 | -4.9 | -6.0 | -8.4 | -10.7 | -12.4 |
| 6 | -5.8 | -6.8 | -8.8 | -11.8 | -12.5 |
| 7 | -5.9 | -7.1 | -9.2 | -12.0 | -12.6 |

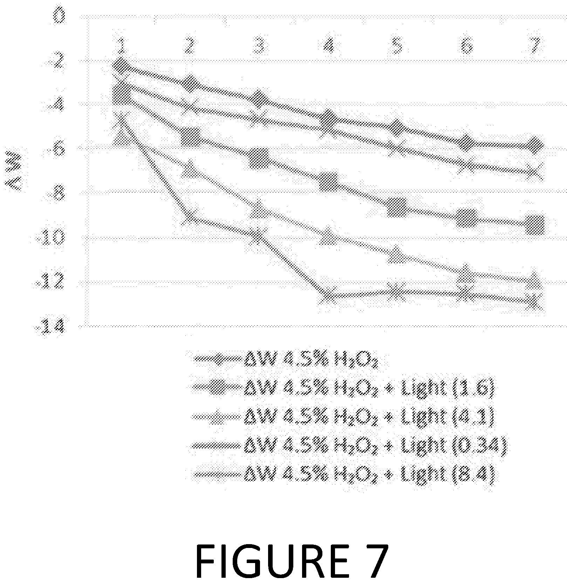

As demonstrated by Table 3, whitening treatment using a combination of $H_2O_2$ and light from the LED at a wavelength of 405 nm can achieve optimal whitening at irradiance densities as low as about 4 $mW/cm^2$. Furthermore, using a irradiance density of about 8 $mW/cm^2$ allows the user to achieve optimal whitening with fewer treatments in that by the fourth treatment, the amount of whitening began to reach the limit of about 12.5, which subsequent treatments at 8 $mW/cm^2$ surprisingly providing no further benefit. Stated otherwise, at about 8 $mW/cm^2$ there is surprisingly a plateau of the whitening effect because even though the power was doubled from 4 $mW/cm^2$ to 8 $mW/cm^2$, the resulting whitening effect was not. The data of Table 3 is represented in graphical form in Figure 7.

Example 3

Example 3 demonstrates the difference in irradiance density of the light emitted from the LED as well as $H_2O_2$ (HP) concentration of the whitening agent. However, for the purposes of the following examples, the $\Delta W$ values have been multiplied by negative one (-1), thereby resulting in a positive value for increases in whiteness. Bovine teeth were whitened according to a plurality of whitening treatments - each treatment included a whitening agent and irradiating a tooth with light from an LED for a irradiation time period of 10 minutes, and the light emitted from the LED having a wavelength of 410 nm.

**Table 4**

|  | 1 | 2 | 3 | 4 | 5 | 6 | 7 | 8 | 9 |
|---|---|---|---|---|---|---|---|---|---|
| 3 wt. % HP (5 $mW/cm^2$) | 3.44 | 6.15 | 8.43 | 10.29 | 11.60 | 12.89 | 13.92 | 14.55 | 15.19 |
| 3 wt. % HP (10 $mW/cm^2$) | 5.83 | 9.44 | 12.26 | 14.02 | 14.78 | 15.48 | - | - | - |

(continued)

| | 1 | 2 | 3 | 4 | 5 | 6 | 7 | 8 | 9 |
|---|---|---|---|---|---|---|---|---|---|
| 6 wt. % HP (No Light) | 2.02 | 3.53 | 4.70 | 5.45 | 6.16 | 6.88 | 7.68 | 8.18 | 8.80 |
| 6 wt. % HP (10 mW/cm$^2$) | 6.58 | 10.66 | 12.94 | 14.21 | 15.17 | 15.68 | 16.10 | 16.61 | 16.75 |
| 9 wt. % HP (No Light) | 2.40 | 3.99 | 5.25 | 6.40 | 7.24 | 8.37 | 9.39 | 9.86 | 10.71 |
| 9 wt. % HP (8 mW/cm$^2$) | 7.77 | 12.42 | 15.01 | 16.29 | 17.03 | 17.77 | 18.33 | 18.59 | 18.96 |

As demonstrated by Table 4, whitening treatment using a combination of $H_2O_2$ and light from the LED at a wavelength of 410 nm can achieve optimal whitening at irradiance density within a range extending from about 3 mW/cm$^2$ to about 10 mW/cm$^2$. Achieving the desired amount of whitening while reducing the amount of $H_2O_2$ in the whitening agent can be achieved by an offset increase in irradiance density, or, alternatively, by increasing the number of treatments. Using the tooth whitening performance set forth in Table 4 allows for the superior tooth whitening while minimizing the time of irradiation, intensity of irradiation, or both - thereby providing a safer product for the user.

Example 4

Example 4 demonstrates the difference in irradiance density and time for whitening agent having 9 wt. % of $H_2O_2$ (HP) concentration. Specifically, bovine teeth were whitened according to a plurality of whitening treatments - each treatment included a whitening agent and irradiating a tooth surface with light from an LED for an irradiation time period of 10 minutes, and the light emitted from the LED having a wavelength of 410 nm.

**Table 5**

| | 1 | 2 | 3 | 4 | 5 | 6 | 7 | 8 |
|---|---|---|---|---|---|---|---|---|
| 30 Min. (No Light) | 4.94 | 7.92 | 10.12 | 11.87 | 13.44 | 14.80 | 14.95 | 16.87 |
| 5 Min. (8mW/cm$^2$) | 4.31 | 8.25 | 10.82 | 13.06 | 14.83 | 16.13 | 17.27 | 18.04 |
| 10 Min. (8mW/cm$^2$) | 7.77 | 12.42 | 15.01 | 16.29 | 17.03 | 17.77 | 18.33 | 18.60 |

As demonstrated by Table 5, whitening treatment using a combination of 9 wt. % $H_2O_2$ and light from the LED at a wavelength of 410 nm can achieve optimal whitening at irradiance density of about 8 mW/cm$^2$ with an irradiation time period ranging from about 5 to about 10 minutes.

Example 5

Example 5 demonstrates the difference in irradiance density and time for whitening agent having 6 wt. % of $H_2O_2$ (HP) concentration. Specifically, bovine teeth were whitened according to a plurality of whitening treatments - each treatment included a whitening agent and irradiating a tooth surface with light from an LED, the light having a wavelength of 410 nm.

**Table 6**

| | 1 | 2 | 3 | 4 | 5 | 6 | 7 | 8 | 9 |
|---|---|---|---|---|---|---|---|---|---|
| 10 Min. (No Light) | 2.02 | 3.53 | 4.70 | 5.45 | 6.16 | 6.88 | 7.68 | 8.18 | 8.80 |
| 30 Min. (No Light) | 3.49 | 5.38 | 7.13 | 8.42 | 9.51 | 10.58 | 11.58 | 12.53 | 13.21 |
| 5 Min. (8mW/cm$^2$) | 4.31 | 8.25 | 10.82 | 13.06 | 14.83 | 16.13 | 17.27 | 18.04 | - |
| 10 Min. (8mW/cm$^2$) | 7.77 | 12.42 | 15.01 | 16.29 | 17.03 | 17.77 | 18.33 | 18.60 | - |

As demonstrated by Table 6, whitening treatment using a combination of 6 wt. % $H_2O_2$ and light from the LED at a wavelength of 410 nm can achieve optimal whitening at irradiance density of about 8 mW/cm$^2$ to 9 mW/cm$^2$ with an irradiation time period ranging from about 5 to about 10 minutes.

Example 6

Example 6 demonstrates the difference in a pulsating light source versus a continuous light source that irradiates a tooth during the whitening process of the current invention. Specifically, bovine teeth were whitened according to a plurality of whitening treatments - each treatment included a whitening agent and irradiating a tooth surface with light from an LED for an irradiation time period of 10 minutes, and the light emitted from the LED having a wavelength of 410 nm. Pulsating vs. non-pulsating LED emitting light at wavelength of 410 nm, the light at the LED having a irradiance density of 8 mW/cm$^2$ and teach treatment period lasting for 10 minutes. The whitening agent that comprises $H_2O_2$ at a concentration of 9 wt. % based on the total weight of the whitening agent.

**Table 7**

|  | 1 | 2 | 3 | 4 | 5 | 6 | 7 | 8 |
|---|---|---|---|---|---|---|---|---|
| No Light | -2.40 | -3.99 | -5.25 | -6.40 | -7.24 | -8.37 | -9.39 | -9.86 |
| Pulsating (1kHz) | -5.07 | -8.71 | -11.29 | -13.46 | -14.89 | -15.95 | -16.58 | -17.27 |
| Continuous | -7.77 | -12.42 | -15.01 | -16.29 | -17.03 | -17.77 | -18.33 | -18.60 |

As demonstrated by Table 7, at a wavelength of 410 nm, better tooth whitening was achieved with light emitted continuously from the LED as compared to the tooth whitening achieved with pulsating light emitted from the LED.

Example 7

Example 7 is directed to a method for bleaching stains - specifically coffee stains. A coffee stain solution was prepared by mixing 3 mL of a buffer solution with 30 µL of a corresponding stain sample. The buffer solution consists of 0.5M phosphate buffer at a pH of 7.1. Each of the coffee stain solution further contain 4.5 wt. % $H_2O_2$ as the whitening agent.
The coffee stain solution was then placed into a sample holder, and the sample holder that was coupled to a stir plate. The coffee stain sample was then irradiated at an area of 1 cm$^2$ (out of 3 cm$^2$) while the saint solution was stirred to create homogenous irradiation of the solution. The wavelength of the light used to irradiate the coffee stain solution was controlled using a spectrofluorometer. The wavelengths were varied by 10 nm increments, ranging from 390 nm to 500 nm at an irradiance density of 1 mW/cm2 +/- 0.5. The results for the bleaching of the coffee stain solution are provided in Table 8.

**Table 8**

| Bleaching Efficacy Coffee | | | | |
|---|---|---|---|---|
| | Minutes of bleaching | | | |
| ($\lambda$) nm | 1 | 5 | 10 | 15 |
| 390 | 19.62% | 34.56% | 35.78% | 38.19% |
| 400 | 11.19% | 25.98% | 34.71% | 39.41% |
| 410 | 10.92% | 24.80% | 32.53% | 37.78% |
| 420 | 8.88% | 23.80% | 32.21% | 37.42% |
| 430 | 9.02% | 22.35% | 30.41% | 34.63% |
| 440 | 8.44% | 22.66% | 29.31% | 34.10% |
| 450 | 9.93% | 23.43% | 31.71% | 36.31% |
| 460 | 8.55% | 20.40% | 27.29% | 31.82% |
| 470 | 7.16% | 21.11% | 29.76% | 35.42% |
| 480 | 5.63% | 16.07% | 21.26% | 28.13% |
| 490 | 3.83% | 13.88% | 19.86% | 24.06% |
| 500 | 3.79% | 13.21% | 17.73% | 20.97% |

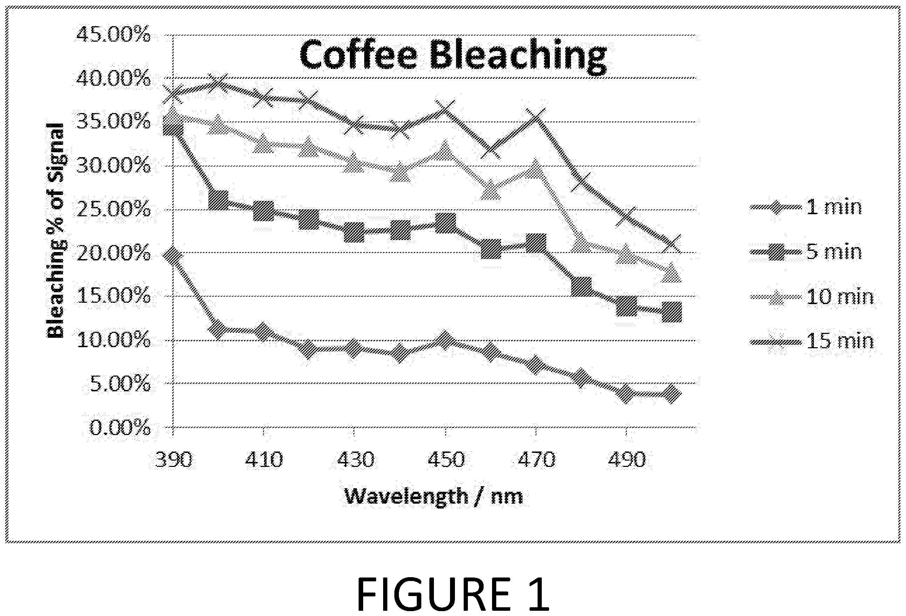

The data presented in Table 8 has been graphically represented in Figure 1.

Example 8

Example 8 is directed to a method for bleaching stains - specifically tea stains. A tea stain solution was prepared by mixing 3 mL of a buffer solution with 30 $\mu$L of a corresponding stain sample. The buffer solution consists of 0.5M phosphate buffer at a pH of 7.1. The tea stain sample contained at least one stain causing organic compounds, such as thearubigins and theaflavins. The tea stain solution further contain 4.5 wt. % $H_2O_2$ as the whitening agent.

The tea stain solution was placed into a sample holder, and the sample holder that was coupled to a stir plate. The tea stain sample was then irradiated at an area of 1 cm$^2$ (out of 3 cm$^2$) while the tea saint solution was stirred to create homogenous irradiation of the solution. The wavelength of the light used to irradiate the tea stain solution was controlled using a spectrofluorometer. The wavelengths were varied by 10 nm increments, ranging from 390 nm to 500 nm at an irradiance density of 1 mW/cm2 +/- 0.5. The results for the bleaching of the tea stain solution are provided in Table 9.

**Table 9**

| Bleaching Efficacy Tea | | | | |
|---|---|---|---|---|
| | Minutes of bleaching | | | |
| ($\lambda$) nm | 1 | 5 | 10 | 15 |
| 390 | 15.41% | 22.76% | 25.25% | 31.29% |
| 400 | 8.23% | 18.70% | 23.99% | 27.87% |
| 410 | 5.18% | 14.00% | 21.13% | 25.35% |
| 420 | 1.64% | 5.99% | 11.23% | 15.05% |
| 430 | 0.38% | 2.75% | 6.63% | 10.09% |
| 440 | 0.28% | 3.57% | 8.56% | 14.02% |
| 450 | 0.40% | 4.65% | 11.21% | 17.02% |
| 460 | 0.57% | 3.25% | 10.04% | 16.95% |
| 470 | 0.60% | 5.55% | 13.62% | 21.06% |
| 480 | 2.48% | 0.11% | 4.63% | 15.37% |
| 490 | -0.21% | 2.57% | 7.32% | 16.97% |
| 500 | 1.77% | -1.06% | 0.30% | 1.43% |

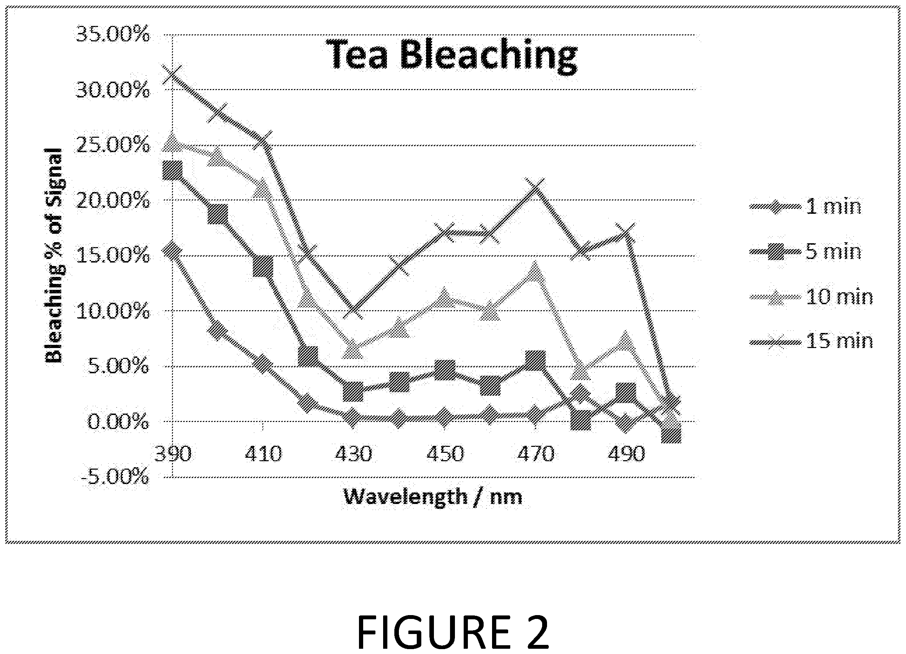

The data presented in Table 9 has been graphically represented in Figure 2.

Example 9

Example 9 is directed to a method for bleaching stains - specifically wine stains. A wine stain solution was prepared by mixing 3 mL of a buffer solution with 30 $\mu$L of a corresponding stain sample. The buffer solution consists of 0.5M phosphate buffer at a pH of 7.1. The wine stain sample contained stain causing organic compounds comprising at least one of anthocyanins, flavonols, flavanols, and hydroxycinnamic acids. The wine stain solution further comprised 4.5 wt. % $H_2O_2$ as the whitening agent.

The wine stain solution was placed into a sample holder, and the sample holder that was coupled to a stir plate. The wine stain sample was then irradiated at an area of 1 cm$^2$ (out of 3 cm$^2$) while the wine saint solution was stirred to create homogenous irradiation of the solution. The wavelength of the light used to irradiate the wine stain solution was controlled using a spectrofluorometer. The wavelengths were varied by 10 nm increments, ranging from 390 nm to 500 nm at an irradiance density of 1 mW/cm2 +/- 0.5. The results for the bleaching of the wine stain solution are provided in Table 10.

**Table 10**

| Bleaching Efficacy Wine | | | | |
|---|---|---|---|---|
| | Minutes of bleaching | | | |
| (λ) nm | 1 | 5 | 10 | 15 |
| 390 | 4.27% | 14.05% | 15.81% | 16.49% |
| 400 | 2.55% | 8.48% | 13.91% | 17.02% |
| 410 | 3.87% | 8.33% | 11.62% | 13.85% |
| 420 | 2.60% | 6.94% | 9.94% | 11.89% |
| 430 | 2.52% | 5.75% | 9.02% | 11.46% |
| 440 | 1.68% | 4.14% | 6.21% | 8.24% |
| 450 | 0.51% | 1.36% | 3.36% | 5.31% |
| 460 | 2.51% | 2.79% | 3.62% | 5.99% |
| 470 | 0.28% | 1.35% | 4.45% | 7.30% |
| 480 | 5.89% | 4.37% | 8.87% | 12.76% |
| 490 | -1.09% | 1.41% | 3.95% | 6.70% |
| 500 | -1.46% | 1.47% | 4.34% | 8.39% |

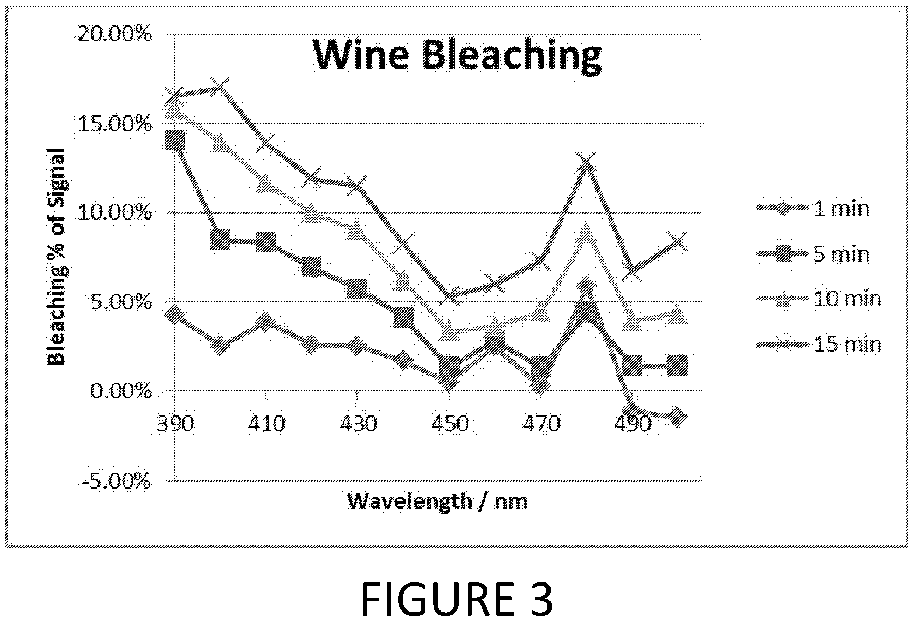

The data presented in Table 10 has been graphically represented in Figure 3.

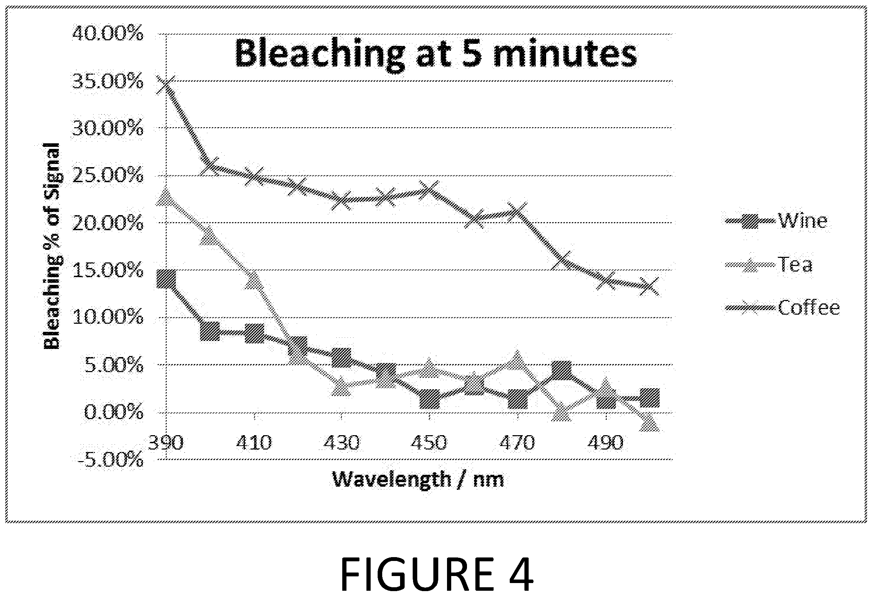

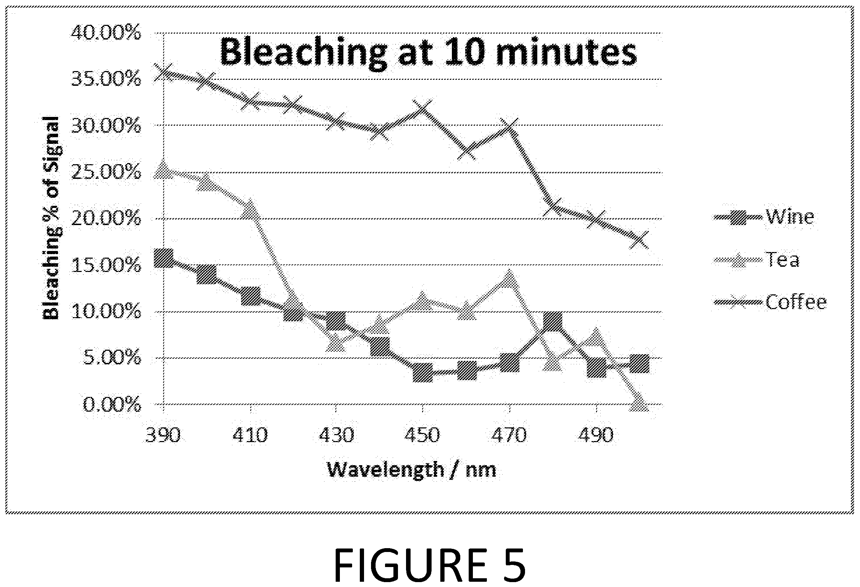

Using the data of Tables 8, 9, and 10, the bleaching characteristics of the tea, coffee, and wine stain samples have been combined at treatment times of 5 minutes and 10 minutes. The combined results for the 5 minute treatment time are presented in Figure 4. The combined results for the 5 minute treatment time are presented in Figure 5.

As shown in Figures 1-5, enhanced bleaching of stain compositions occurs at wavelengths approaching the UV spectrum - i.e. from 390 to 410 nm. Bleaching efficacy drops as the wavelength exceeds 410 nm. As demonstrated by both Figures 4 and 5, it has also been surprisingly discovered that enhanced bleaching characteristics can be achieved within the wavelength range of 450 nm to 490 nm - as there is an unexpected increase in bleaching of each stain composition.

Furthermore, as demonstrated in Figure 6, the bovine teeth bleaching boost performance that is summarized in Table 2 may be superimposed on the stain data shown in Figures 1-5 to establish that the bovine teeth whitening data essentially correlates with the stain bleaching data. Specifically, both the bovine teeth whitening and the stain bleaching data demonstrate two peaks in whitening / bleaching performance: the first being at 390 nm to 420 nm and the second being at 450 to 490 nm. Thus, the tea, coffee, and wine data may be correlated to bovine teeth whitening performance data allowing the calculation of a lower wavelength threshold for the stain bleaching data when applied to bovine teeth. Thus, based on the previously discussed inferences, some embodiments of the present invention further comprise a whitening agent for use in a method of bleaching stains of coffee, tea, and/or wine from a tooth using a light source that is operated at a wavelength ranging from 400 to 410 nm.

**Claims**

1. A tooth whitening agent comprising hydrogen peroxide in a concentration ranging from 2 wt. % to 10 wt. % based on the total weight of the tooth whitening agent for use in a method of whitening teeth comprising:

    a) applying a tooth whitening agent to the tooth, for a pretreatment prior to
    b) continuously irradiating a tooth with light generated by a light source for an irradiation time period, which ranges from 60 seconds to 1800 seconds, the light having a wavelength that ranges from 400 nm to 410 nm and the light source emitting the light at an irradiance density ranging from 3 mW/cm$^2$ to 10.0 mW/cm$^2$.

2. The whitening agent for use in the method according to claim 1, wherein the wavelength is 405 nm.

3. The whitening agent for use according to claim 1 wherein step b) is performed while the tooth whitening agent is on the tooth.

**4.** The whitening agent for use according to any one of claims 1 to 3, wherein the whitening agent is substantially free of zinc oxide.

**5.** The whitening agent for use according to any one of claims 1 to 4, wherein the hydrogen peroxide is present in a concentration ranging from 3 wt. % to 6 wt. % based on the total weight of the whitening agent.

**6.** The whitening agent for use according to any one of claims 1 to 5, wherein the irradiation time period ranges from 300 seconds to 600 seconds.

**7.** The whitening agent for use according to any one of claims 1 to 6, wherein the irradiation density is measured at an exterior surface of the light source, and wherein the exterior surface of the light source is spaced from the tooth by a distance ranging from about 2 mm to about 12 mm.

**8.** The whitening agent for use according to any one of claims 1 to 7, the method comprising:
in step

b) irradiating a tooth with light emitted from a light source for an irradiation time period, wherein the light has a wavelength that ranges from 400 nm to 410 nm and the irradiation time period ranges from 300 seconds to 1800 seconds; and
c) upon expiration of the irradiation time period, ceasing to irradiate the tooth with the light for a recovery time period, thereby completing a treatment cycle.

**9.** The whitening agent for use according to claim 8, wherein the treatment cycle is repeated 3 to 8 times and the recovery time period is at least 4 hours.

**10.** The whitening agent for use according to claims 8 or 9, wherein the irradiation time period is greater than 600 second.

**Patentansprüche**

**1.** Zahnbleichmittel, umfassend Wasserstoffperoxid in einer Konzentration im Bereich von 2 Gew.-% bis 10 Gew.-%, bezogen auf das Gesamtgewicht des Zahnbleichmittels, zur Verwendung in einem Verfahren zum Aufhellen von Zähnen, umfassend:

a) Auftragen eines Zahnbleichmittels auf den Zahn zur Vorbehandlung vor dem
b) kontinuierlichen Bestrahlen eines Zahns mit Licht, das von einer Lichtquelle erzeugt wird, für eine Bestrahlungszeitdauer, die im Bereich von 60 Sekunden bis 1800 Sekunden liegt, wobei das Licht eine Wellenlänge im Bereich von 400 nm bis 410 nm hat und die Lichtquelle das Licht mit einer Bestrahlungsdichte im Bereich von 3 mW/cm$^2$ bis 10,0 mW/cm$^2$ emittiert.

**2.** Bleichmittel zur Verwendung in dem Verfahren nach Anspruch 1, wobei die Wellenlänge 405 nm beträgt.

**3.** Bleichmittel zur Verwendung nach Anspruch 1, wobei Schritt b) durchgeführt wird, während sich das Zahnbleichmittel auf dem Zahn befindet.

**4.** Bleichmittel zur Verwendung nach einem der Ansprüche 1 bis 3, wobei das Bleichmittel im Wesentlichen frei von Zinkoxid ist.

**5.** Bleichmittel zur Verwendung nach einem der Ansprüche 1 bis 4, wobei das Wasserstoffperoxid in einer Konzentration im Bereich von 3 Gew.-% bis 6 Gew.-%, bezogen auf das Gesamtgewicht des Bleichmittels, vorhanden ist.

**6.** Bleichmittel zur Verwendung nach einem der Ansprüche 1 bis 5, wobei die Bestrahlungsdauer zwischen 300 Sekunden und 600 Sekunden liegt.

**7.** Bleichmittel zur Verwendung nach einem der Ansprüche 1 bis 6, wobei die Bestrahlungsdichte an einer Außenfläche der Lichtquelle gemessen wird und wobei die Außenfläche der Lichtquelle in einem Abstand von etwa 2 mm bis etwa 12 mm von dem Zahn entfernt ist.

8. Bleichmittel zur Verwendung nach einem der Ansprüche 1 bis 7, wobei das Verfahren umfasst:
im Gleichschritt

b) Bestrahlen eines Zahns mit Licht, das von einer Lichtquelle für eine Bestrahlungszeitdauer emittiert wird, wobei das Licht eine Wellenlänge im Bereich von 400 nm bis 410 nm hat und die Bestrahlungszeitdauer im Bereich von 300 Sekunden bis 1800 Sekunden liegt; und
c) nach Ablauf der Bestrahlungszeit wird die Bestrahlung des Zahns mit dem Licht für eine Erholungszeit eingestellt, wodurch ein Behandlungszyklus abgeschlossen wird.

9. Bleichmittel zur Verwendung nach Anspruch 8, wobei der Behandlungszyklus 3 bis 8 Mal wiederholt wird und die Erholungszeit mindestens 4 Stunden beträgt.

10. Bleichmittel zur Verwendung nach Anspruch 8 oder 9, wobei die Bestrahlungsdauer größer als 600 Sekunden ist.

**Revendications**

1. Agent de blanchiment des dents comprenant du peroxyde d'hydrogène à une concentration allant de 2 % en poids à 10 % en poids par rapport au poids total de l'agent de blanchiment des dents pour une utilisation dans un procédé de blanchiment des dents comprenant :

a) l'application d'un agent de blanchiment des dents sur la dent, pour un pré-traitement avant
b) l'irradiation en continu d'une dent avec de la lumière générée par une source de lumière pendant une période de temps d'irradiation, qui va de 60 secondes à 1 800 secondes, la lumière ayant une longueur d'onde qui va de 400 nm à 410 nm et la source de lumière émettant la lumière à une densité d'éclairement allant de 3 mW/cm$^2$ à 10,0 mW/cm$^2$.

2. Agent de blanchiment pour une utilisation dans le procédé selon la revendication 1, dans lequel la longueur d'onde est de 405 nm.

3. Agent de blanchiment pour une utilisation selon la revendication 1, dans lequel l'étape b) est réalisée alors que l'agent de blanchiment des dents est sur la dent.

4. Agent de blanchiment pour une utilisation selon l'une quelconque des revendications 1 à 3, dans lequel l'agent de blanchiment est sensiblement exempt d'oxyde de zinc.

5. Agent de blanchiment pour une utilisation selon l'une quelconque des revendications 1 à 4, dans lequel le peroxyde d'hydrogène est présent à une concentration allant de 3 % en poids à 6 % en poids par rapport au poids total de l'agent de blanchiment.

6. Agent de blanchiment pour une utilisation selon l'une quelconque des revendications 1 à 5, dans lequel la période de temps d'irradiation est comprise entre 300 secondes et 600 secondes.

7. Agent de blanchiment pour une utilisation selon l'une quelconque des revendications 1 à 6, dans lequel la densité d'éclairement est mesurée au niveau d'une surface extérieure de la source de lumière, et dans lequel la surface extérieure de la source de lumière est espacée de la dent d'une distance allant d'environ 2 mm à environ 12 mm.

8. Agent de blanchiment pour une utilisation selon l'une quelconque des revendications 1 à 7, le procédé comprenant :
à l'étape

b) l'irradiation d'une dent avec de la lumière émise par une source de lumière pendant une période de temps d'irradiation, la lumière ayant une longueur d'onde qui va de 400 nm à 410 nm et la période de temps d'irradiation va de 300 secondes à 1 800 secondes ; et
c) à l'expiration de la période de temps d'irradiation, la cessation de l'irradiation de la dent avec la lumière pendant une période de temps de récupération, achevant ainsi un cycle de traitement.

9.  Agent de blanchiment pour une utilisation selon la revendication 8, dans lequel le cycle de traitement est répété 3 à 8 fois et la période de temps de récupération est d'au moins 4 heures.

10. Agent de blanchiment pour une utilisation selon les revendications 8 ou 9, dans lequel la période de temps d'irradiation est supérieure à 600 secondes.

FIGURE 1

FIGURE 2

FIGURE 3

EP 3 367 963 B1

FIGURE 4

FIGURE 5

EP 3 367 963 B1

FIGURE 6

EP 3 367 963 B1

EP 3 367 963 B1

FIGURE 7

## REFERENCES CITED IN THE DESCRIPTION

**Patent documents cited in the description**

- US 2015064645 A1